# FASCICULE DE BREVET EUROPEEN

(11) **EP 0 814 763 B1**
(45) Date de publication et mention de la délivrance du brevet: **03.07.2002**
(21) Numéro de dépôt: 96908162.9
(22) Date de dépôt: 21.03.1996
(51) Int. Cl.: A61K 7/48, A61K 35/78

(54) **COMPOSITION DERMATOLOGIQUE ET/OU COSMETIQUE DEPIGMENTANTE**
HAUTBLEICHENDE DERMATOLOGISCHE UND/ODER KOSMETISCHE ZUSAMMENSETZUNG
DEPIGMENTING DERMATOLOGICAL AND/OR COSMETIC COMPOSITION

(30) Priorité: 23.03.1995 FR 9503425
(43) Date de publication de la demande: 07.01.1998
(73) Titulaire: Pierre Fabre Dermo-Cosmetique, 92100 Boulogne (FR)
(72) Inventeur: TARROUX, Marie-Christine, F-31300 Toulouse (FR); NAVARRO, Roger, F-09100 Pamiers (FR); MSIKA, Philippe, F-31000 Toulouse (FR); FABRE, Bernard, F-31450 Belberaud (FR)
(74) Mandataire: Ahner, Francis
(86) Numéro de dépôt international: FR9600422
(87) Numéro de publication internationale: WO9629050

(56) Documents cités:
- EP-A- 0 307 277
- FR-A- 2 150 208

## Description

La présente invention se rapporte à l'utilisation d'une composition contenant un extrait actif obtenu à partir de piloselle pour la fabrication d'un médicament dépigmentant. L'invention a également pour objet l'utilisation d'une composition cosmétique contenant un extrait actif obtenu à partir de piloselle pour réduire et/ou supprimer les tâches de pigmentation ainsi qu'une méthode de traitement cosmétique pour réduire et/ou supprimer les tâches de pigmentation.

La couleur de la peau est due à plusieurs substances : l'hémoglobine des vaisseaux, les carotènoïdes du derme et, surtout, la mélanine de l'épiderme.

Cette mélanine est produite par les mélanocytes de la couche basale, sous l'action de la lyrosinase, du cuivre et de l'oxygène.

Sous l'effet de stimulations exogènes ou endogènes, il peut apparaître des modifications de la teinte de la peau. Ce sont les dyschromies.

La modification du pigment cutané peut se faire:
. soit par excès : l'hyperchromie,
. soit par défaut : l'hypochromie.

Elle peut siéger dans l'épidémie ou dans le derme et être due à une variation de la quantité de mélanine ou du nombre de mélanocytes.

Les hyperchromies sont des accumulations de pigments mélaniques, de caroténoïdes ou de pigments exogènes.

La mélanine de la peau est formée par une association complexe d'Eumélanine et de Phéomélanine.

Leurs biosynthèses sont communes jusqu'à la dopaquinone (double oxydation de la tyrosine par la tyrosinase, enzyme cupro-protéique). Leur chemin ensuite diverge.

L'Eumélanine brune est un polymère d'indole-5-6-quinone tandis que la Phéomélanine responsable de la couleur rousse est un composé contenant près de 10% de soufre et de structure polymère de la cystéinyl dopa.

D'autres enzymes que la tyrosinase participent à la génèse et au contrôle des mélanines :
* la Dopachrome oxydo-réductase : qui transforme la dopachrome en 5-6-dihydroxyindole et contrôle la mélanogénèse en absence de tyrosinase,
* l'α-Glutamyl transpepsidase transformerait le glutathion dopa en cystéinyl dopa.
* le système Glutathion (réductase-péroxydase) qui régirait le carrefour entre la biosynthèse de l'Eumélanine ou de la Phéomélanine.

Il semblerait que le taux de soufre environnant soit l'élément déterminant d'une telle orientation.

L'activité glutathion réductase et le taux de glutathion réduit sont plus élevés chez les sujets roux que chez les sujets bruns.
* la Dopachrome tautomérase régule la réaction dopachrome acide 5-6 dihydroxyindole-2 carboxylique et contrôle la proportion de sous-unités carboxylées dans le polymère mélanique.

Les agents dépigmentants ou blanchissants du teint sont des composés chimiques capables d'agir à l'échelon tissulaire, cellulaire ou subcellulaire.

Ils agissent sur la mélanine elle-même ou sur l'existence de mélanocyte (mélanocytotoxicité).

Les modes d'action généraux peuvent être les suivants :
. Inhibition de la formation des mélanosomes,
. Altération de la structure des mélanosomes,
. Inhibition de la biosynthèse de la tyrosinase,
. Inhibition de la biosynthèse de la mélanine,
. Interférence du transfert des mélanosomes vers les kératinocytes;
. Effet chimique sur la mélanine avec augmentation de la dégradation des mélanosomes dans les kératinocytes.

D'autre part, il est nécessaire de mettre en évidence et de supprimer le facteur induisant l'hyperpigmentation avant de la traiter (U.V., parfum, oestroprogestatif) et de conseiller une protection solaire type protection maximale tout au long du suivi médical).

Les motivations qui poussent à décolorer la peau peuvent être diverses.

L'éclaircissement franc du teint est recherché en Afrique Noire avec des solutions traditionnelles ou chimiques qui présentent des effets secondaires néfastes importants sur l'aspect et la structure de la peau.

La pâleur ou la blancheur du visage asiatique est obtenue avec des molécules agissant avec le moins de toxicité possible (l'arbutine, l'acide kojique, l'acide ascorbique).

Le traitement des taches d'hyperpigmentation des sujets blancs fait appel à des molécules diverses dont la principale, l'hydroquinone, fait l'objet d'une surveillance accrue et dont la dose maximale en cosmétique est de 2%.

Il existe donc un besoin pour des compositions présentant une activité dépigmentante marquée à des concentrations modérées, et qui soient bien tolérées par la peau.

C'est pourquoi la présente invention a pour objet l'utilisation d'une composition contenant un extrait actif obtenu à partir de piloselle pour la fabrication d'un médicament dépigmentant ainsi que l'utilisation d'une composition cosmétique contenant un extrait actif obtenu à partir de piloselle pour réduire et/ou supprimer les tâches de pigmentation.

La Piloselle, Hieracium pilosella, appartient à la famille des Composées. C'est une petite plante herbacée, gazonnante de 10 à 30 cm de hauteur.

Les feuilles forment une rosette basale, elles sont longues, ovales, blanchâtres en dessous, hérissées de soie sur les deux faces.

La tige florifère est dressée, unique, et porte un capitule à fleurs hermaphrodites, toutes ligulées, à 5 dents et d'un jaune clair. Les bractées sont nombreuses et portent souvent des poils glanduleux noirs.

Le fruit est un akène à aigrettes, finement côtelé.

La Piloselle fleurit de Mai à Septembre.

On la trouve dans les lieux secs, dans presque toute l'Europe, dans le nord de l'Afrique et en Amérique du Nord. Elle est très commune en France mais rare dans la région méditerranéenne.

De nombreuses études chimiques effectuées sur la plante ont permis d'isoler et d'identifier un grand nombre de molécules. La Piloselle est caractérisée par la présence d'oxycoumarines : ombelliférone (hydroxy-7 coumarine) et ombelliférone-7-monoglucoside ou skimmine. La teneur en ombelliférone varie selon les organes, les feuilles sont les plus riches et selon les saisons ; la teneur maximale est en été, minimale à la fin de l'hiver.

Des acides phénoliques ont également été identifiés ainsi que de flavonoïdes : l'apigénine et ses dérivés, la lutéoline et ses dérivés. Certains auteurs notent la présence de tanins dans toute la plante.

La Piloselle a été utilisée pour ses propriétés diurétiques en médecine traditionnelle. Ses propriétés cholérétiques et cholagogues ont également été décrites ; ainsi FR 2 549 373 utilise l'essence de Piloselle pour la préparation d'une composition favorisant la digestion et la sécrétion biliaire. Le brevet FR 744 M l'a proposée comme hypocholestérolémiant. Des compositions aqueuses contenant du silanol et des extraits de Piloselle ont été proposées dans FR 2 620 029 pour le traitement superficiel des vaisseaux lymphatiques.

De manière inattendue, la Demanderesse a maintenant mis en évidence que des principes actifs pouvant être préparés à partir de Piloselle possédent des propriétés pouvant avantageusement être mises à profit dans des compositions dermocosmétiques ; la Demanderesse a mis en évidence l'activité dépigmentante d'un extrait de Piloselle.

Un extrait actif peut notamment être obtenu par un procédé qui comprend une étape d'extraction à partir des parties aériennes et/ou racines de piloselle, par un solvant polaire choisi dans le groupe comprenant l'eau, l'alcool, l'acétone et leurs mélanges ; dans ces solvants hydroalcooliques et/ou hydroacétonique, la proportion de chacun des constituants peut varier entre 0 et 100.

Selon l'un des modes de réalisation de l'invention, la plante est broyée, puis extraite par un alcool de C₁ à C₄ ou par un mélange eau/alcool de C₁ à C₄ ou par l'acétone ou par un mélange eau/acétone, dans des proportions eau/alcool C₁ à C₄ ou eau/acétone variant de 100/0 à 0/100. Le rapport plante/solvant varie de 1/5 à 1/20.

De préférence, la plante entière est broyée puis extraite par un alcool de C₁ à C₄ ou par un mélange eau-alcool de C₁ à C₄, dans des proportions variant de 90/10 à 10/90.

L'extraction peut se faire statiquement ou sous agitation, à des températures allant de l'ébullition à la température ambiante. La durée de l'extraction se situe entre 1 heure et 24 heures. Après extraction, les solutions sont récupérées par filtration ou par essorage.

On peut procéder, dans un second temps, à des étapes d'enrichissement en principes actifs.

Les alcools ou l'acétone sont alors évaporés sous vide, à des températures situées entre 40° C et 100° C. Les solutions aqueuses concentrées sont purifiées par extraction liquide/liquide.

Les solvants organiques utilisés sont des solvants type alcanes, comme l'hexane, l'heptane, des solvants chlorés tels dichlorométhane, le chloroforme, le butanol, l'acétate d'éthyle ou des solvants dont la miscibilité avec l'eau dépend de la force ionique comme l'acétone, l'isopropanol, l'éthanol. Dans ce cas, la solution aqueuse sera saturée en (NH₄)₂SO₄, Na Cl ou Na₂SO₄. Le pH de l'extraction liquide/liquide peut être ajusté entre pH 2,5 et pH 8 selon les solvants d'extraction.

Les phases organiques sont récupérées et filtrées.

L'extrait peut être utilisé tel quel. On peut également évaporer le solvant organique sous vide, à une température située entre 40° C et la température d'ébullition du solvant. L'extrait sec récupéré est alors broyé sous forme de poudre.

Les différents extraits de piloselle, sont dosés pour leur teneur en ombelliférone, par chromalographie liquide haute performance. Le dosage est effectué sur une colonne C₁₈ avec une phase mobile : eau-propanol-tétrahydrofurane-acide phosphorique : 95 - 5 - 15 - 0,5 par rapport à un témoin pur d'ombelliférone.

Les teneurs varient selon le degré de purification de l'extrait. Ainsi, les proportions en ombelliférone par rapport à la matière sèche d'un extrait non purifié, varient de 0,5 à 2%, pour un extrait purifié de 4 à 10%.

L'invention comprend également l'utilisation d'un produit de composition analogue à l'extrait de piloselle, mais qui peut être préparé par synthèse.

De préférence, l'extrait de Piloselle est présent avec un titre en ombelliférone compris entre 0,05% et 10% en poids de la composition totale.

Les compositions selon l'invention peuvent se présenter sous forme de lotions, émulsions, crèmes, onguents, pommades etc. Elles contiennent en outre tous les excipients de formulation connus de l'homme du métier appropriés à une bonne application topique. Elles contiennent notamment des stabilisants, conservateurs, tensioactifs, parfums, colorants.

Dans les compositions selon l'invention, l'extrait actif de piloselle peut être associé à des composés kératolytiques, par exemple l'acide salicylique, l'acide lactique, glycolique, malique ainsi que d'autres acides α hydroxylés connus de l'homme du métier.

Selon un autre aspect de l'invention les compositions dermocosmétiques décrites précédemment contiennent également au moins un autre principe actif dépigmentant et/ou au moins un filtre ou un écran solaire; de tels filtres minéraux et/ou organiques sont connus de l'homme du métier qui adaptera leur choix et leurs concentrations en fonction du degré de protection recherché. L'invention a également pour objet une méthode de traitement cosmétique, caractérisée en ce qu'on applique localement un extrait de piloselle.

Plus particulièrement, l'invention a pour objet l'utilisation dans une méthode de traitement cosmétique d'un extrait actif de Piloselle pour réduire et/ou supprimer les tâches de pigmentation.

Les pigmentations apparaissent souvent sous les effets des rayonnements UVA et B, lors d'expositions prolongées au soleil.

La Demanderesse a donc évalué les activités filtres UV et piégeurs de radicaux libres des extraits de Piloselle. Ces activités vont contribuer à l'activité dépigmentante des extraits de Piloselle.

L'un des aspects de l'invention concerne donc l'utilisation dans une méthode cosmétique d'un extrait de Piloselle comme filtre anti-U.V. A et U.V. B. Elle conceme également l'utilisation d'un extrait de Piloselle comme substance anti-radicalaire.

Les propriétés dépigmentantes des extraits de Piloselle ou d'un produit actif susceptible d'être préparé à partir de Piloselle peuvent, selon un autre aspect de l'invention, conduire à l'utilisation d'un produit actif susceptible d'être obtenu à partir de Piloselle pour la préparation d'un médicament actif comme dépigmentant

Les compositions pharmaceutiques contiendront l'extrait de piloselle, associé à des excipients pharmaceutiquement acceptables.

Les exemples qui suivent sont destinés à illustrer l'invention sans aucunement en limiter la portée.

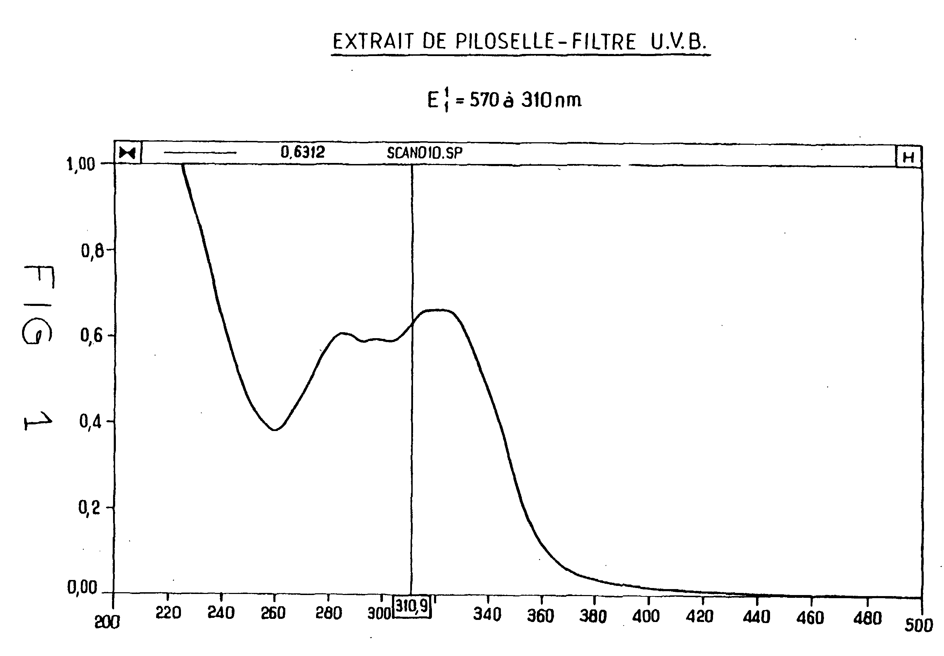

On se réfèrera à la Figure en annexe qui représente l'activité UV d'un extrait de piloselle.

### Exemple 1

100 kg de plantes entières de Piloselle, sont broyés puis extraits par 800 Kg d'éthanol à 20% sous agitation à reflux pendant 1 heure. La solution hydroalcoolique est récupérée par essorage puis filtrée. Elle est ensuite concentrée sous vide à 60° C jusqu'à l'obtention de 100 kg de solution aqueuse.

Le pH de cette solution est amené à pH 3,5 par addition d'acide chlorhydrique.

Une extraction liquide/liquide est réalisée avec 300 kg d'acétate d'éthyle sous agitation pendant 2 heures. Après décantation la phase organique est récupérée, concentrée sous vide à 40° C puis séchée et broyée. On récupère 1 kg d'extrait sec dont la teneur en ombelliférone se situe entre 6 et 8%.

### Exemple 2

1 kg de plantes entières de Piloselle est extrait par 5 kg d'éthanol 60%, à froid, durant 24 heures. La solution hydro-alcoolique est récupérée par filtration. Sa teneur en ombelliférone se situe entre 1 et 2% par rapport à la matière sèche.

### Exemple 3

10 kg de plantes entières de Piloselle sont broyés à l'aide d'un broyeur à marteaux. La poudre de plantes est ensuite extraite à reflux, sous agitation, avec 100 kg d'un mélange méthanol-eau : 50 - 50. La solution est récupérée par essorage puis filtration. Elle est ensuite concentrée, sous vide, à 50° C jusqu'à l'obtention de 10 kg de concentrat aqueux. On ajoute à ce dernier, jusqu'à saturation, du sulfate d'ammonium puis 30 kg d'isopropanol.

Le mélange est agité durant 2 heures puis on laisse décanter. La phase isopropylique est récupérée, concentrée puis séchée sous vide.

On obtient 4,5 kg d'une poudre dont la teneur en ombelliférone varie de 4 à 6%.

### Exemple 4

1 kg de plantes entières de piloselle est broyé, puis extrait par un mélange eau/acétone 80/20 à reflux pendant une heure.

La solution hydroacétonique obtenue après filtration est concentrée, puis séchée sous pression réduite à une température située entre 40 et 50° C. L'extrait sec obtenu est broyé, puis titré entre 5 et 8% d'ombelliférone.

### Exemple 5 : Evaluation de l'activité filtre solaire

L'activité filtre solaire UVA et UVB de l'extrait de Piloselle est évaluée par l'étude spectrophotométrique de l'extrait titré à 5% en ombelliférone.

Cet extrait présente un maximum d'absorption à 325 nm et un coefficient d'extension spécifique de 570 à 310 nm.

Ceci démontre le potentiel filtre UV B de l'extrait de Piloselle. En comparaison, le Parsol MCX, filtre chimique UV B présente un coefficient d'extension spécifique de 810 à 310 nm.

Les résultats sont représentés sur la figure annexée à la demande.

### Exemple 6 : Evaluation de l'activité antiradicalaire

La réaction radicalaire se divise en 3 étapes successives :
l'initiation, la propagation et la disparition des radicaux libres :
   . l'initiation est due à l'anion superoxyde O₂- qui apparaît sous l'effet de facteurs tels que le rayonnement UV ou le stress,
   . la propagation est le fait des radicaux hydroxylés OH,
la disparition des radicaux libres. Les piégeurs de radicaux libres agissent sur l'anion superoxyde O₂- mais également sur les radicaux hydroxyls. Aussi, nous avons cherché à évaluer l'activité antiradicalaire sur ces deux étapes.

L'activité sur l'anion superoxyde est réalisée par test in vitro. L'anion superoxyde est généré par photo-oxydation radicalaire, par sensibilisation de la riboflavine au rayonnement visible.

L'indicateur coloré utilisé est le nitrobleu de tétrazolium, électrophile qui est réduit par l'anion superoxyde généré en diformazan.

L'activité antiradicalaire de l'extrait de Piloselle est exprimée par la concentration en extrait qui inhibe 50% de l'activité réductrice de l'anion superoxyde sur le NBT, la CI₅₀. Pour un extrait de Piloselle titré à 5% en ombelliférone, la CI₅₀ est de 0,3 mg/ml.

L'activité sur la propagation radicalaire, donc sur les radicaux hydroxyls, est évaluée sur le DPHH, diphényl-picryl-hydrazyl-hydrate, radical libre stable coloré. La CI₅₀ d'un extrait de Piloselle contenant 5% d'ombelliférone est de 0,025 mg/ml. Dans ce test, la vitamine E a une CI₅₀ de 0,006 mg/ml.

### Exemple 7 : Evaluation de l'activité dépigmentante in vitro

In vitro, on recherche une activité inhibitrice sur l'activité de la tyrosinase, enzyme majeure du processus de pigmentation. En présence d'oxygène, la réaction de la tyrosinase sur de la tyrosine, se traduit par une augmentation de la densité optique du milieu de réaction à 280 nm.

Toute inhibition de l'action de la tyrosinase sur la tyrosine directe sur l'enzyme ou indirecte par compétition avec la tyrosine, se concrétise par une augmentation moins importante de la densité optique à 280 nm.

On calcule la CI 50, concentration d'extrait de Piloselle inhibant 50% du signal de réaction de la tyrosinase.

Pour un extrait de Piloselle dosé à 5% en ombelliférone, la CI 50 est de 30 µg/ml.

### Exemple 8 : Dépigmentation in vivo

L'évaluation de l'activité dépigmentante consiste à rechercher un effet inhibiteur de préparations topiques contenant un extrait de Piloselle titré à 5% en ombelliférone sur l'hyperpigmentation de l'épiderme caudal, induite par exposition aux rayonnements Ultra-Violets chez la souris pigmentée.

La préparation topique est appliquée 7 jours sur 7, pendant 6 semaines consécutives, les irradiations aux rayonnements UV 5 jours sur 7 pendant 42 jours.

Les animaux font l'objet d'un examen quotidien afin de surveiller les modifications de pigmentation et d'apprécier leur intensité. Les colorations sont attribuées selon une échelle de couleurs.

Les animaux traités avec l'extrait de Piloselle présentent une dépigmentation non homogène par rapport au lot témoin irradié. Cet effet ne peut être attribué à un effet de filtration UV puisque les produits sont appliqués après l'irradiation.

A l'issue des 42 jours de traitement, la peau de l'appendice caudal des animaux est prélevée après leur sacrifice. Une mesure de la densité optique est effectuée sur le prélèvement d'épiderme à 700 nm.

On observe une diminution significative de la densité optique de l'épiderme des animaux traités avec de l'extrait de Piloselle. Cette diminution traduit l'effet dépigmentant de cet extrait.

Enfin, la répartition et la quantification de mélanine dans les couches de l'épiderme sont évaluées par analyse d'image.

On observe une diminution globale de la mélanine de 24% qui se retrouve aussi bien au niveau des basales que des couches superficielles de l'épiderme des souris traitées par l'extrait de Piloselle.

### Exemple 9 : Formules dépigmentantes

Les formules suivantes peuvent être données à titre d'exemple :

| **A- Lotion dépigmentante** | |
|---|---|
| Extrait de Piloselle titré à 4% | 10 g |
| Propylène glycol | 20 g |
| Alcool éthylique | 10 g |
| Eau distillée qsp | 100 g |

L'extrait de Piloselle est dissous dans le mélange Propylène glycol-Alcool éthylique, puis complété par l'eau distillée.

| **B- Gel aqueux** | |
|---|---|
| Extrait de Piloselle titré à 8% | 5 g |
| Propylène glycol | 20 g |
| Alcool éthylique | 15 g |
| Hydroxy Propyl cellulose | 1,5 g |
| Eau distillée qsp | 100 g |

L'extrait de Piloselle est dissous dans le mélange Propylène glycol-Alcool éthylique, puis complété par l'eau distillée.

La Cellulose est introduite sous agitation jusqu'à formation du gel.

| **C- Emulsion** | |
|---|---|
| Evtrait de Piloselle titré à 4% | 3 g |
| Ester de Glucose (Glucate SS) | 4 g |
| Ester de Glucose éthoxylé (Glucate SSE 20) | 4 g |
| Huile de Vaseline épaisse | 10 g |
| Triglycérides C₈ - C₁₀ | 4 g |
| Cyclométhicone (Dow Corning 345) | 4 g |
| Polymère carboxyvinylique (Carbomer 940) | 1 g |
| EDTA, 2 Na | 0,2 g |
| Eau distillée qsp | 100 g |

### Exemple 10 : Roll On au serum dépigmentant

| | |
|---|---|
| Extrait de Pilosse | 0,1 à 10% |
| Alcool à 95° | 35 à 75% |
| Adipate d'Isopropyle | 5 à 15% |
| Acide oléique | 0,01 à 1% |
| Propylène glycol | 10 à 40% |
| Klucel MF | 0,1 à 2% |
| Eau qsp | 100 |

### Exemple 11 : Sérum dépigmentant/antiradicalaire/kératolytique

| | |
|---|---|
| Extrait de Piloselle | 0,1 à 10% |
| Acide salycilique | 0,1 à 5% |
| Vitamine C PCA | 0,1 à 10% |
| Hydroquinone | 0,1 à 5% |
| Alcool batylique | 0,05 à 1% |
| Alcool à 95° | 10 à 75% |
| Adipate d'Isopropyle | 5 à 15% |
| Acide oléique | 0,01 à 1% |
| Propylène glycol | 5 à 40% |
| Klucel MF | 0,1 à 2% |
| Eau qsp | 100 |

### Exemple 12 : Crème de jour dépigmentante

| | |
|---|---|
| Arlacel 165 (ICI) | 5 à 15% |
| Alcool cétylique | 0,1 à 3% |
| Acide stéarique | 1 à 6% |
| Huile de paraffine | 2 à 15% |
| Isostéarate d'Isopropyle | 1 à 6% |
| Huile de Tournesol | 1 à 2% |
| Extrait de Piloselle | 0,1 à 25% |
| Alcool batylique | 0,05 à 1% |
| A.H.A. | 0,1 à 25% |
| Propylène glycol | 0,1 à 10% |
| Eau qsp | 100 |

### Exemple 13 : Roll On Dépigmentant H/E

| | |
|---|---|
| Arlamol E | 0,1 à 10% |
| Brij 72 | 1 à 3% |
| Brij 721 | 1 à 5% |
| Extrait de Piloselle | 0,1 à 25% |
| Acide oléique | 0,05 à 1% |
| Eau qsp | 100 |
| | |
| Alcool | 0 à 10% |

### Exemple 14 : Lait dépigmentant H/E

| | |
|---|---|
| Arlatone 985 | 1 à 5% |
| Brij 721 | 1 à 3% |
| Miglyol 812 | 1 à 10% |
| Arlamol MD | 1 à 10% |
| Extrait de Piloselle | 0,1 à 25% |
| Atlas G 2330 | 0,1 à 5% |
| Acide salycilique | 0,1 à 2% |
| Alcool | 0 à 10% |
| α Bisabolol | 0,05 à 0,5% |
| Vitamine C Phosphate magnesium | 0,1 à 3% |
| Eau qsp | 100 |

### Exemple 15 : Emulsion hydratante et dépigmentante protectrice des UVB et A

| | |
|---|---|
| Extrait de Piloselle | 0,1 à 25% |
| Arlacel 2121 | 1 à 10% |
| Arlamol HD | 1 à 10% |
| Alcool | 0 à 10% |
| Acide oléique | 0,1 à 1% |
| Alcool béhénylique | 0,1 à 2% |
| Acétate de Tocophérol | 0,05 à 1% |
| Glycérine | 0,01 à 10% |
| A.H.A. | 0,1 à 25% |
| Vitamine C PCA | 0,1 à 3% |
| Glycérine | 0,1 à 15% |
| TIO₂ | 0 à 25% |
| ZnO | 0 à 25% |
| Cinnamate | 0 à 10% |
| Dibenzoylméthane | 0 à 4% |
| Eau qsp | 100 |

### Exemple 16 : Crème de nuit dépigmentante émulsion E/H

| | |
|---|---|
| Arlacel 481 | 2 à 10% |
| Paraffine | 1 à 20% |
| Glycérine | 1 à 15% |
| Mg SO₄ | 0,5 |
| Eau qsp | 100 |
| Extrait de Piloselle | 0,1 à 25% |

### Exemple 17 : Emulsion nutritive dépigmentante

| | |
|---|---|
| Arlacel 1689 | 1 à 5% |
| Miglyol 812 | 1 à 15% |
| Aérosil 972 | 0,1 à 0,5% |
| Glycérine | 1 à 15% |
| MgSO₄ | 0,1 à 0,5% |
| Eau qsp | 100 |
| Extrait de Piloselle | 0,1 à 25% |
| Alcool bacylique | 0,05 à 0,3% |

### Exemple 18 : Stick dépigmentant

| | |
|---|---|
| Super Hartolan | 9% |
| Lunacera Alba | 4,5% |
| Lunacera C 40 | 7,2% |
| Lunacera C 46 | 3,7% |
| Lunacera M | 4,5% |
| Vaseline | 0 à 18% |
| Huile de Ricin | 0 à 28% |
| Isopropyl Myristate | 0 à 30% |
| TIO₂ - Mica (Timica Silk blue) | 0,1 à 5% |
| TIO₂ Ultrafin | 0 à 25% |
| ZnO Ultrafin | 0 à 25% |
| Extrait de Piloselle | 0,1 à 25% |
| Alcool batylique | 0,05 à 1% |
| β Carotène | 0,005 à 0,1% |
| Abil WE 09 | 0,1 à 2% |
| | |
| Eau qsp | 0 à 5% |

### Exemple 19 : Emulsion sprau ultrafine dépigmentante

| | |
|---|---|
| Alcool béhénique éthoxylé (Mergital B 10) | 5 à 10% |
| Huile Végétale | 0,1 à 5% |
| Lanette 22 | 0,1 à 2% |
| Extrait de Piloselle | 0,1 à 25% |
| Vitamine C Palmitate | 0,1 à 3% |
| A.H.A. | 0,1 à 25% |
| α Bisabolol | 0,05 à 0,5% |
| Eau qsp | 100 |

## Revendications

1. Utilisation d'une composition contenant un extrait actif obtenu à partir de piloselle pour la fabrication d'un médicament dépigmentant.

2. Utilisation selon la revendication 1, **caractérisée en ce que** l'extrait actif est obtenu après une étape d'extraction par un solvant choisi dans le groupe comprenant l'eau, l'alcool, l'acétone, et leurs mélanges en toutes proportions, à partir des parties aériennes et/ou des racines de piloselle.

3. Utilisation selon la revendication 2, **caractérisée en ce que** l'extrait actif est obtenu après une étape d'extraction hydroalcoolique, avec un rapport eau/alcool compris entre 90/10 et 10/90, à partir des parties aériennes et/ou des racines de piloselle.

4. Utilisation selon l'une des revendications 1 à 3, **caractérisée en ce que** la composition contient un mélange d'un extrait de Piloselle avec au moins un autre principe actif dépigmentant.

5. Utilisation selon l'une des revendications 1 à 4, **caractérisée en ce que** la composition contient en outre au moins un filtre solaire organique ou minéral.

6. Utilisation selon l'une des revendications 1 à 5, **caractérisée en ce que** la composition contient également au moins un principe actif kératolytique.

7. Utilisation selon l'une des revendications 1 à 6, **caractérisée en ce que** l'extrait de Piloselle est présent avec un titre en ombelliférone compris entre 0,05% et 10% de la composition totale.

8. Utilisation non thérapeutique d'une composition cosmétique contenant un extrait actif obtenu à partir de piloselle pour réduire et/ou supprimer les tâches de pigmentation.

9. Utilisation d'une composition cosmétique selon la revendication 8, **caractérisée en ce que** l'extrait actif est obtenu après une étape d'extraction par un solvant choisi dans le groupe comprenant l'eau, l'alcool, l'acétone et leurs mélanges en toutes proportions, à partir des parties aériennes et/ou des racines de piloselle.

10. Utilisation d'une composition cosmétique selon la revendication 9, **caractérisée en ce que** l'extrait de actif est obtenu après une étape d'extraction hydroalcoolique, avec un rapport eau/alcool compris entre 90/10 et 10/90, à partir des parties aériennes et/ou des racines de piloselle.

11. Utilisation d'une composition cosmétique selon l'une des revendications 8 à 10, **caractérisée en ce qu'**elle contient un mélange d'un extrait de Piloselle avec au moins un autre principe actif dépigmentant.

12. Utilisation d'une composition cosmétique selon l'une des revendications 8 à 11, **caractérisée en ce qu'**elle contient en outre au moins un filtre solaire organique ou minéral.

13. Utilisation d'une composition cosmétique selon l'une des revendications 8 à 12, **caractérisée en ce qu'**elle contient également au moins un principe actif kératolytique.

14. Utilisation d'une composition cosmétique selon l'une des revendications 8 à 13, **caractérisée en ce que** l'extrait de Piloselle est présent avec un titre en ombelliférone compris entre 0,05% et 10% de la composition totale.

15. Méthode de traitement cosmétique pour réduire et/ou supprimer les tâches de pigmentation, **caractérisée en ce qu'**on applique localement un extrait de piloselle.

16. Méthode de traitement cosmétique selon la revendication 15, **caractérisée en ce que** l'on applique localement un extrait de piloselle comma filtre anti UVA et B.

17. Méthode de traitement cosmétique selon la revendication 15 ou 16, **caractérisée en ce que** l'on applique localement un extrait de piloselle comme substance anti-radicalaire.

## Patentansprüche

1. Verwendung einer Zusammensetzung, die einen aktiven Extrakt enthält, der ausgehend von Kleinem Habichtskraut erhalten wird, für die Herstellung eines depigmentierenden Medikaments.

2. Verwendung nach Anspruch 1, **dadurch gekennzeichnet, dass** der aktive Extrakt nach einem Extraktionsschritt durch ein Lösungsmittel, das ausgewählt ist aus der Gruppe umfassend Wasser, Alkohol, Aceton und deren Mischungen in allen Verhältnissen, ausgehend von den Luftteilen und/oder den Wurzeln von Kleinem Habichtskraut erhalten wird.

3. Verwendung nach Anspruch 2, **dadurch gekennzeichnet, dass** der aktive Extrakt nach einem wässrig-alkoholischen Extraktionsschritt mit einem Verhältnis Wasser/Alkohol von 90/10 bis 10/90 ausgehend von den Luftteilen und/oder den Wurzeln von Kleinem Habichtskraut erhalten wird.

4. Verwendung nach einem der Ansprüche 1 bis 3, **dadurch gekennzeichnet, dass** die Zusammensetzung eine Mischung eines Extrakts von Kleinem Habichtskraut mit mindestens einem anderen depigmentierenden Wirkstoff enthält.

5. Verwendung nach einem der Ansprüche 1 bis 4, **dadurch gekennzeichnet, dass** die Zusammensetzung darüber hinaus mindestens einen organischen oder mineralischen Sonnenfilter enthält.

6. Verwendung nach einem der Ansprüche 1 bis 5, **dadurch gekennzeichnet, dass** die Zusammensetzung auch mindestens einen keratolytischen Wirkstoff enthält.

7. Verwendung nach einem der Ansprüche 1 bis 6, **dadurch gekennzeichnet, dass** der Extrakt von Kleinem Habichtskraut mit einem Titer an Umbelliferon von 0,05% bis 10% der Gesamt-Zusammensetzung vorliegt.

8. Nicht-therapeutische Verwendung einer kosmetischen Zusammensetzung, die einen aktiven Extrakt enthält, der ausgehend von Kleinem Habichtskraut erhalten wird, um Pigmentierungsflecken zu verringern und/oder zu unterdrücken.

9. Verwendung einer kosmetischen Zusammensetzung nach Anspruch 8, **dadurch gekennzeichnet, dass** der aktive Extrakt nach einem Extraktionsschritt durch ein Lösungsmittel, das ausgewählt ist aus der Gruppe umfassend Wasser, Alkohol, Aceton und deren Mischungen in allen Verhältnissen, ausgehend von den Luftteilen und/oder den Wurzeln von Kleinem Habichtskraut erhalten wird.

10. Verwendung einer kosmetischen Zusammensetzung nach Anspruch 9, **dadurch gekennzeichnet, dass** der aktive Extrakt nach einem wässrig-alkoholischen Extraktionsschritt mit einem Verhältnis Wasser/Alkohol von 90/10 bis 10/90 ausgehend von den Luftteilen und/oder den Wurzeln von Kleinem Habichtskraut erhalten wird.

11. Verwendung einer kosmetischen Zusammensetzung nach einem der Ansprüche 8 bis 10, **dadurch gekennzeichnet, dass** sie eine Mischung eines Extrakts von Kleinem Habichtskraut mit mindestens einem anderen depigmentierenden Wirkstoff enthält.

12. Verwendung einer kosmetischen Zusammensetzung nach einem der Ansprüche 8 bis 11, **dadurch gekennzeichnet, dass** sie darüber hinaus mindestens einen organischen oder mineralischen Sonnenfilter enthält.

13. Verwendung einer kosmetischen Zusammensetzung nach einem der Ansprüche 8 bis 12, **dadurch gekennzeichnet, dass** sie auch mindestens einen keratolytischen Wirkstoff enthält.

14. Verwendung einer kosmetischen Zusammensetzung nach einem der Ansprüche 8 bis 13, **dadurch gekennzeichnet, dass** der Extrakt von Kleinem Habichtskraut mit einem Titer an Umbelliferon von 0,05 % bis 10 % der Gesamt-Zusammensetzung vorliegt.

15. Verfahren zur kosmetischen Behandlung, um Pigmentierungsflecken zu verringern und/oder zu unterdrücken, **dadurch gekennzeichnet, dass** man lokal einen Extrakt von Kleinem Habichtskraut aufträgt.

16. Verfahren zur kosmetischen Behandlung nach Anspruch 15, **dadurch gekennzeichnet, dass** man lokal einen Extrakt von Kleinem Habichtskraut als Anti-UVA- und -B-Filter aufträgt.

17. Verfahren zur kosmetischen Behandlung nach Anspruch 15 oder 16, **dadurch gekennzeichnet, dass** man lokal einen Extrakt von Kleinem Habichtskraut als Antiradikal-Substanz aufträgt.

## Claims

1. Use of a composition containing an active extract obtained from mouse-ear hawkweed, for the manufacture of a depigmenting medicinal product.

2. Use according to Claim 1, **characterized in that** the active extract is obtained after a step of extraction with a solvent chosen from the group comprising water, alcohol, acetone, and mixtures thereof in all proportions, from the aerial parts and/or the roots of mouse-ear hawkweed.

3. Use according to Claim 2, **characterized in that** the active extract is obtained after a step of aqueous-alcoholic extraction, with a water/alcohol ratio of between 90/10 and 10/90, from the aerial parts and/or the roots of mouse-ear hawkweed.

4. Use according to one of Claims 1 to 3, **characterized in that** the composition contains a mixture of an extract of mouse-ear hawkweed with at least one other depigmenting active principle.

5. Use according to one of Claims 1 to 4, **characterized in that** the composition also contains at least one organic or inorganic sunscreen.

6. Use according to one of Claims 1 to 5, **characterized in that** the composition also contains at least one keratolytic active principle.

7. Use according to one of Claims 1 to 6, **characterized in that** the extract of mouse-ear hawkweed is present with an umbelliferone titre of between 0.05% and 10% of the total composition.

8. Non-therapeutic use of a cosmetic composition containing an active extract obtained from mouse-ear hawkweed, for reducing and/or eliminating pigmentations marks.

9. Use of a cosmetic composition according to Claim 8, **characterized in that** the active extract is obtained after a step of extraction by a solvent chosen from the group comprising water, alcohol, acetone and mixtures thereof in all proportions, from the aerial parts and/or the roots of mouse-ear hawkweed.

10. Use of a cosmetic composition according to Claim 9, **characterized in that** the active extract is obtained after a step of aqueous-alcoholic extraction with a water/alcohol ratio of between 90/10 and 10/90, from the aerial parts and/or the roots of mouse-ear hawkweed.

11. Use of a cosmetic composition according to one of Claims 8 to 10, **characterized in that** it contains a mixture of an extract of mouse-ear hawkweed with at least one other depigmenting active principle.

12. Use of a cosmetic composition according to one of Claims 8 to 11, **characterized in that** it also contains at least one organic or inorganic sunscreen.

13. Use of a cosmetic composition according to one of Claims 8 to 12, **characterized in that** it also contains at least one keratolytic active principle.

14. Use of a cosmetic composition according to one of Claims 8 to 13, **characterized in that** the extract of mouse-ear hawkweed is present with an umbelliferone titre of between 0.05% and 10% of the total composition

15. Cosmetic treatment method for reducing and/or eliminating pigmentation marks, **characterized in that** an extract of mouse-ear hawkweed is applied locally.

16. Cosmetic treatment method according to Claim 15, **characterized in that** an extract of mouse-ear hawkweed is applied locally as an anti-UVA and anti-UVB screening agent.

17. Cosmetic treatment method according to Claim 15 or 16, **characterized in that** an extract of mouse-ear hawkweed is applied locally as a free-radical-scavenging substance.
